(19) Europäisches Patentamt
European Patent Office
Office européen des brevets

(11) **EP 4 134 961 A1**

(12) **EUROPEAN PATENT APPLICATION**
published in accordance with Art. 153(4) EPC

(43) Date of publication:
**15.02.2023 Bulletin 2023/07**

(51) International Patent Classification (IPC):
**G16B 40/20** (2019.01)　　　**G16B 30/10** (2019.01)
**G16B 50/00** (2019.01)

(21) Application number: **20924030.8**

(22) Date of filing: **02.12.2020**

(86) International application number:
**PCT/KR2020/017475**

(87) International publication number:
**WO 2021/182718 (16.09.2021 Gazette 2021/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **12.03.2020 KR 20200030835**

(71) Applicant: **Infoboss Co., Ltd.
Seoul 06088 (KR)**

(72) Inventors:
• **PARK, Jong-Sun
　Seoul 06088 (KR)**
• **SON, Jang-Hyuk
　Seoul 06088 (KR)**

(74) Representative: **Nordmeyer, Philipp Werner
df-mp Dörries Frank-Molnia & Pohlman
Patentanwälte Rechtsanwälte PartG mbB
Theatinerstraße 16
80333 München (DE)**

(54) **BIOCHEMICAL PATHWAY EXPANSION METHOD, DEVICE AND PROGRAM**

(57)　A method of expansion of a biochemical pathway includes: inputting an input pathway including a starting substance, an intermediate substance, and a terminating substance; investigating analogs having a similar function and a different structure to a substance of each step on the input pathway; establishing an association relationship between the analogs for another pathway including the analogs; and performing a recalculation of a changed intermediate substance and a calculation of a new terminating substance, for a step after the establishing of the association relationship.

FIG.1

EP 4 134 961 A1

**Description**

[Technical Field]

The present invention relates to a method, an apparatus, and a program of expansion of a biochemical pathway, and more particularly, to a method, an apparatus, and a program that generate and display the new pathway (expanded pathway) of which substances in a given biochemical pathway (hereinafter, abbreviated as 'pathway') (input pathway) are replaced with analogs and provide the probability of existence of final secondary metabolites of the expanded pathway.

[Background Technology]

By the rapid development of biological and computer sciences, various biochemical data have been accumulated as a digitalized form. Accordingly, the time and cost as well as the high accuracy for manipulating and utilizing the digitalized biochemical data became the most critical factors.

Usually, sequencing is the method to decipher nucleotide bases of the DNA which organisms have. There are two major sequencing methods: one is Sanger Sequencing Method, which is a classical method, and the other is Next Generation Sequencing (NGS) Method which has been commercialized since 2005.

Sanger Sequencing Method which can generate around 1,000 basepairs per time requires relatively high costs (around 4 won per basepair). However, Sequencing by Synthesis (SBS) method, which is one of NGS technologies, had been established around 2000s when large scale of sequencing projects, such as Human Genome Project (HGP), were in progress. Currently, SBS method provides extremely low cost in comparison to the Sanger Sequencing Method: around 3 million won per 120Gbp (0.000025 won per basepair).

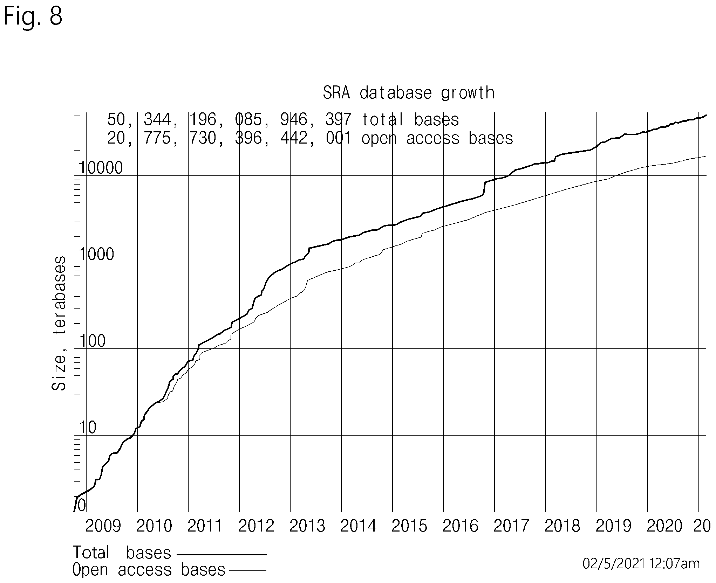

Owing to this NGS technologies, many genome projects and related research have been activated, resulting in huge amount of nucleotide data generated by NGS. As of 2019, 31 Pbp (31,067,314,313,468,958 bp) sequences were deposed in the NCBI Short Read Archive (SRA; https://trace.ncbi.nlm.nih.gov/Traces/sra/) (FIG. 8).

The major motivation to reduce the time and cost of sequencing is the activated genome sequencing and related projects by rapid spread of these NGS technologies. For example, the cost for generating NGS raw reads of one individual human genome is around 1.2 million won (90 Gbp raw reads in Korea).

In general, it is known that plant genome is usually longer and more complex than those of animal species so that it requires more time and cost to decipher their genomes. However, NGS technologies have been successfully applied to plant genomes resulting in a large amount of plant genomes. As of February 2020, 1,986 plant genomes originated from 638 species are available (Plant Genome Database Release 2.6; http://www.plantgenome.info/).

Beside them, Genome-wide association study (GWAS), Genotype by sequencing (GBS), hybrid sequencing (HybSeq), and organelle genome sequencing also utilize NGS technologies. NGS raw reads generated by these methods are not sufficient or not suitable to assemble genomes, but cover broad range of species or individuals in comparison to the typical genome projects. It indicates that these data have potential to be utilized once the proper methods for rescuing genomes are established.

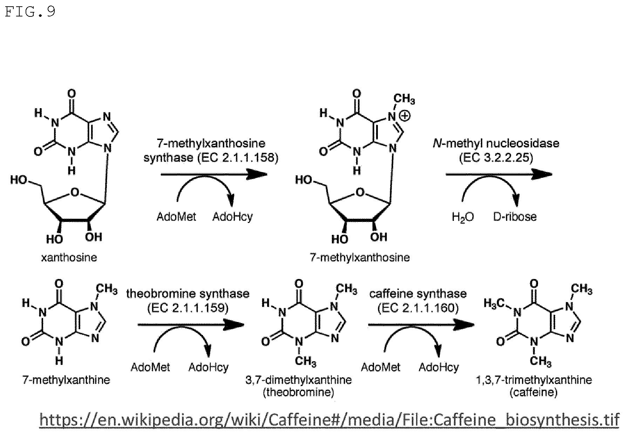

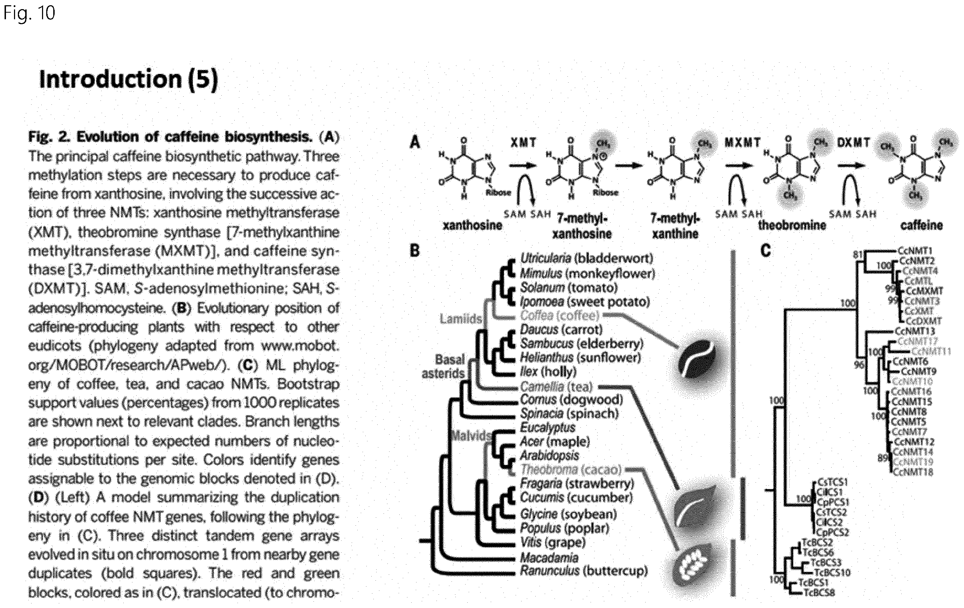

On the other hand, secondary metabolites originated from plant resources have been utilized in various ways. For example, Illicium verum, raw materials of Tamiflu®, generates a lot of shikimic acids as secondary metabolites, which is one of antiviral compounds. Tamiflu® was developed and released in the market based on these secondary metabolites. In addition, caffeine found in the leaf or fruits of coffee (*Coffea arabica*) and tea (*Camellia sinensis*) has been world-widely utilized as a beverage. These secondary metabolites were synthesized through the biochemical pathways in plants and major pathways have been well identified. For example, caffeine is synthesized from xanthosine by four steps (FIG. 9). Moreover, the enzymes involved in the four steps of caffeine biosynthesis were successfully identified from the whole genome of coffee (*Coffea canephora*) and were compared with the related enzymes from the other plant species (FIG. 10).

There is a critical limitation to predict the biochemical pathways which can generate useful phytocompound based on the known biochemical pathways even though there are many biochemical pathways identified till now. It is because one pathway contains multiple enzymes (see B panel in FIG. 10) and sometimes it is difficult to assign one enzyme to the enzymes in the pathway. Moreover, biochemical pathway itself contains the multiple stages which can be partially utilized by the other pathways, which is another hurdle to analyze the pathways in detail.

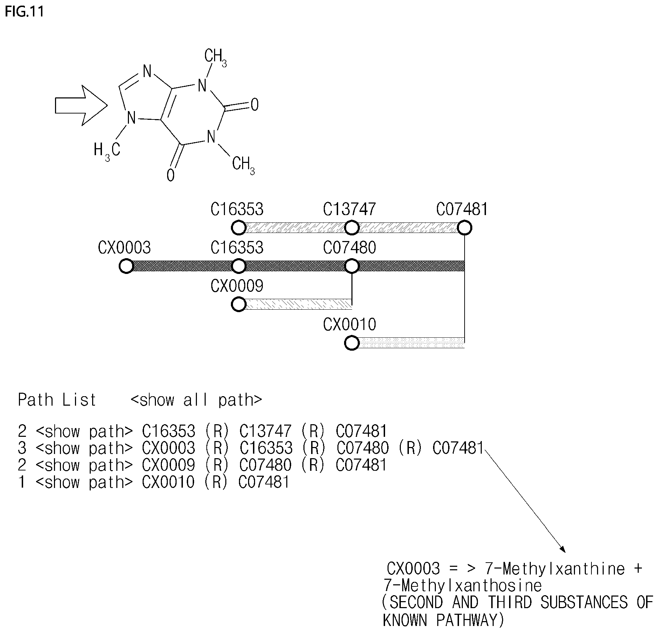

The KEGG (https://www.genome.jp/kegg/) database contains primary and secondary metabolite pathways with providing their complete map. Even in the complete map of known biochemical pathways, the prediction program (https://www.genome.jp/tools/pathpred/) predicts four possible but fragmented pathways of caffeine (FIG. 11. There are four predicted pathways of caffeine, which is different from the identified pathway (FIG. 9)). It indicates that biochemical pathway is more complex than experts expected, and additional research is strongly required.

On the other hand, it is known that plant species have their own effects based on the Korean Traditional Medicine: e.g., Plantago species has a good effect to female disease and balloon flower (*Platycodon grandiflorum*) is good to bronchial tubes as well as cold. However, it is difficult to determine active compounds with the current techniques and to prepare enough amount of plant species for industrial application.

In the fields of medicine, food, and cosmetics, it is needed to develop new products using secondary metabolites originated from the natural plant resources. However, it is also difficult to identify secondary metabolites and its biochemical pathways from plants. In addition, it is also cost and time-consuming tasks. The patent, entitled 'Systems for Predicting Complex Traits associated genes in plants using an *Arabidopsis* gene network' (Patent Document 1), describes the method to predict genes related to specific features based on gene network of *Arabidopsis thaliana.* The prediction result of the patent is the input data of the present invention. The present invention predicts various secondary metabolites as final products by identifying and expanding the related biochemical pathways in plants and assigning enzymes to these biochemical pathways, so that this patent is not related to the present invention.

The patent entitled 'Method and device for selecting pathway of a target compound' (Patent Document 2) describe the way to determine an optimized pathway for synthesis. It may be methodologically related to the parts of the present invention, 'a methodology for finding an optimal pathway in reconstruction' or 'expansion of possible pathways.' However, the present invention aims for predicting all possible but reasonable pathways considering with features of chemical reactions and biochemical synthesis. Taken together, the patent document 2 is not related to the current invention.

[Related Art Document]

[Patent Document]

   (Patent Document 1) Korean Patent Gazette No. 10-1568399

   (Patent Document 2) Korean Patent Laid-Open Publication Gazette No. 10-2017-0095711

[Non-Patent Document]

Denoeud, F., Carretero-Paulet, L., Dereeper, A., Droc, G., Guyot, R., Pietrella, M., Zheng, C., Alberti, A., Anthony, F., Aprea, G. and Aury, J.M., 2014. The coffee genome provides insight into the convergent evolution of caffeine biosynthesis. science, 345(6201), pp.1181-1184.

[Disclosure]

[Technical Problem]

The present invention provides a technology of evaluating an operation of discovering enzyme genes related to pathways using a lot of plant genomic information currently and predicting a role of the enzyme genes systematically and expression-probabilistically in order to overcome limitations of the conventional pathway prediction technology as described above.

In addition, the present disclose provides a technology of deriving specific substances (secondary metabolite) generated by each of the unknown plants from plant genomic information, securing and providing inferable generation mechanism (pathway) information and applying the information to various industrial fields such as new drug development, health food, and cosmetics development, and reducing enormous time and cost to achieve a lot of development in industrial application of plant resources.

In addition, the present discloses provides a technology of receiving a pathway, and replacing substances for each step in the input pathway with analogs and then tracking a reaction of the next steps to generate and present an expanded pathway for the input pathway.

In addition, the present invention provides a technology of calculating and presenting a substance difference between substances in each step in an input pathway and substances in each corresponding step in an expanded pathway corresponding thereto.

In addition, the present invention provides a technology of calculating and presenting a substantiality score of an expanded pathway by determining pathway substantiality of an expanded pathway from a substantiality score of an input pathway and a substance difference between pathways.

In addition, the present invention provides a technology of selecting and presenting a high-scoring expanded pathway by performing substantiality filtering by a substantiality score for an expanded pathway.

In addition, the present invention provides a technology of predicting and presenting a calculation probability of a secondary metabolite as a terminating substance by an expanded pathway by weighting and calculating substances in each step in the expanded pathway.

[Technical Solution]

According to an aspect of the present invention, a method of expansion of a biochemical pathway includes: inputting an input pathway including a starting substance, an intermediate substance, and a terminating substance; investigating analogs having a similar function and a different structure to a substance of each step on the input pathway; establishing an association relationship between the analogs for another pathway including the analogs; and performing a recalculation of a changed intermediate substance and a calculation of a new terminating substance, for a step after the establishing of the association relationship.

Here, the method of expansion of a biochemical pathway may further include: extracting a high-scoring expanded pathway having a score higher than a reference value by substantially filtering the expanded pathway by a substantiality score of the expanded pathway indicating a probability that the expanded pathway actually exists.

In addition, the method of expansion of a biochemical pathway may further include: calculating an actual probability of existence of a secondary metabolite which is a terminating substance by the expanded pathway by weighting and calculating each substance of each step in the expanded pathway.

In addition, the method of expansion of a biochemical pathway may further include: calculating a substance difference between the substance of each step in the input pathway and the substance of each corresponding step in the expanded pathway corresponding thereto, in which the substance difference may be defined as (1 - substance similarity), and the substance similarity may be a value of 0 to 1 represented by digitizing the substance similarity.

In addition, the method of expansion of a biochemical pathway may further include: calculating the substantiality score of the expanded pathway by determining the pathway substantiality of the expanded pathway based on the substantiality score of the input pathway and the substance difference of each step between the input pathway and the expanded pathway.

Here, the substantiality score of the input pathway may be calculated depending on the calculation of a fitness score of an enzyme gene in each step of the input pathway.

In addition, when there are a large number of enzymes mapped for each step of the input pathway, a maximum value among the fitness scores of the enzymes may be selected, a sum of the fitness scores selected for all steps of the input pathway may be calculated, and the substantiality score may be calculated by an average value obtained by dividing the sum of the fitness scores by the number of steps of the input pathway.

On the other hand, according to another aspect of the present invention, an apparatus of expansion of a biochemical pathway includes: an input module configured to input an input pathway including a starting substance, an intermediate substance, and a terminating substance; an analog investigation module configured to investigate analogs having a similar function and a different structure to a substance of each step on the input pathway; an analog association module configured to establish an association relationship between the analogs for another pathway including the analogs; and a change substance calculation module configured to perform a recalculation of a changed intermediate substance and a calculation of a new terminating substance, for a step after the establishing of the association relationship.

In addition, a program of expansion of a biochemical pathway recorded in an information processing device readable storage medium to cause the information processing device to execute each step of the method described above.

[Advantageous Effects]

According to the present invention, it is possible to provide the technology of evaluating the operation of discovering the enzyme genes related to the pathways using a lot of plant genomic information currently and predicting the role of the enzyme genes systematically and expression-probabilistically.

In addition, it is possible to provide the technology of deriving the specific substances (secondary metabolite) generated by each of the unknown plants from plant genomic information, securing and providing the inferable generation mechanism (pathway) information and applying the information to various industrial fields such as new drug development, health food, and cosmetics development, and reducing enormous time and cost to achieve a lot of development in the industrial application of plant resources.

In addition, it is possible to provide the technology of receiving the pathway, and replacing substances for each step in the received pathway with analogs and then tracking the reaction of the next steps to generate and present the expanded pathway for the received pathway.

In addition, it is possible to provide the technology of calculating and presenting the substance difference between the substances in each step in the input pathway and the substances in each corresponding step in the expanded pathway corresponding thereto.

In addition, it is possible to provide the technology of calculating and presenting the substantiality score of the expanded pathway by determining the pathway substantiality of the expanded pathway from the substantiality score of the input pathway and the substance difference between the pathways.

In addition, it is possible to provide the technology of selecting and presenting the high-scoring expanded pathway by performing the substantiality filtering by the substantiality score for the expanded pathway.

In addition, it is possible to provide the technology of predicting and presenting the calculation probability of the secondary metabolite as the terminating substance by the expanded pathway by weighting and calculating the substances in each step in the expanded pathway.

[Description of Drawings]

FIG. 1 is a block diagram of a biochemical pathway expansion apparatus to which a biochemical pathway expansion method of present invention is applied.

FIG. 2 is a flowchart of a method of expansion of a biochemical pathway according to the present invention.

FIG. 3 is a structural formula of an analog of alcohol, which is an example of an analog used in the method of expansion of a biochemical pathway according to the present invention.

FIG. 4 is an explanatory diagram illustrating a flow from an input of the input pathway to substantiality scoring of the expanded pathway.

FIG. 5 is a diagram illustrating an action of Monooxygenase, which is an example of an enzyme, in the description of the role of the enzyme.

FIG. 6 is a diagram illustrating an example of predicting a final product in which an OH group is added to a Coumarin substance by the enzyme Monooxygenasedp.

FIG. 7 is a diagram illustrating a flow in which a list of predicted secondary metabolites and an actual probability (score) are calculated through scoring for substances related to the prediction or expanded pathway.

FIG. 8 is a growth state graph illustrating a state in which sequencing data using an NGS technique is accumulated in the NCBI SRA database over time.

FIG. 9 is a diagram illustrating a known pathway of caffeine.

FIG. 10 is a diagram illustrating a partial excerpt from a comparative study of a pathway of coffee caffeine and genes related to tea and cacao.

FIG. 11 is a diagram illustrating a result of a pathway prediction request for caffeine in a pathway prediction service of KEGG database.

<Explanation of Reference Numerals and Symbols>

10:  Input module
20:  Analog investigation module
30:  Analog association module
40:  Change substance calculation module
50:  Substance difference calculation module
60:  Pathway substantiality scoring module
70:  Substantiality filtering module
80:  Secondary metabolite probability prediction module

[Best Mode]

Hereinafter, a method, an apparatus, and a program of reconstructing a biochemical pathway according to the present invention will be described in detail with reference to the accompanying drawings. However, for members having the same function by the same configuration, the same reference numerals are maintained even if the drawings are different, and thus a detailed description thereof may be omitted.

In addition, the relationship in which other members are arranged or connected to front and rear, left and right, and top and bottom of a member includes a case where a separate member is inserted in the middle. Conversely, when a member is said to be 'right' front and rear, left and right, or top and bottom the other member, it means that there is no separate member in the middle. In addition, unless explicitly described to the contrary, "including" other components will be understood to imply the inclusion of other components rather than the exclusion of other components.

Further, terms 'first', 'second', and the like, will be used to distinguish components having the same names from each other, and will not be necessarily limited to a sequence thereof. In addition, terms such as 'unit', 'means', 'part', 'member', and 'module' described in the specification mean a unit of a comprehensive structure that performs at least one function or operation. One constituent unit may be divided into two or more, and conversely, two or more constituent units may be integrated into one and implemented. In addition, information processing devices such as terminals and servers described in the specification basically mean hard wiring, which means hardware in which a specific function or operation is implemented, but should not be construed to be limited to specific hardware, and it does not exclude soft wiring made of software that is driven to implement a specific function or operation on general hardware. That is, the terminal or server may be any apparatus or software, such as app, installed on a certain device.

In addition, the size and thickness of each component illustrated in the drawings are arbitrarily illustrated for convenience of description, so the present invention is not necessarily limited to what is illustrated in the drawings, and in order to clearly express various parts and areas such as layers and areas, the thickness may be exaggerated and enlarged or reduced.

<Basic Configuration-Method>

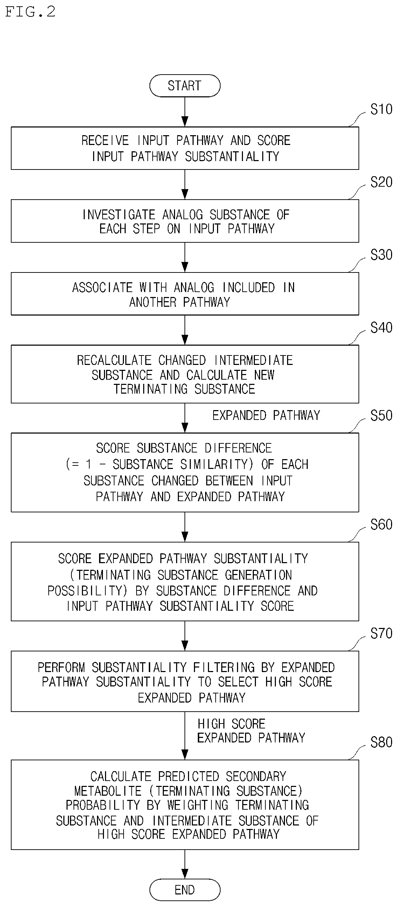

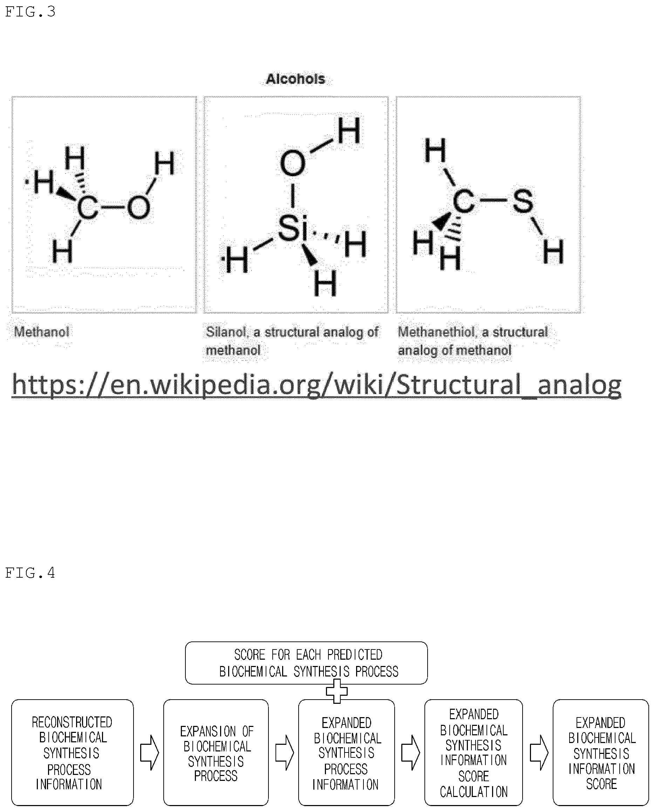

As illustrated in FIG. 2, a method of expansion of a biochemical pathway according to an embodiment of the present invention includes an input step S10, an analog investigation step S20, an analog association step S30, and a change substance calculation step S40. As illustrated in FIG. 4, the method is a process of receiving information on an input pathway and generating information on an expanded pathway through a process of expanding a pathway to be described later. As described later, the information on the expanded pathway may be used together with a substantiality score of an input pathway to be used to represent the substantiality score by the substantiality scoring of the expanded pathway.

The input step S10 is a step of inputting an input pathway including a starting substance, an intermediate substance, and a terminating substance. The input pathway may be a pathway known to produce a specific secondary metabolite as a final terminating substance, an unknown pathway obtained from samples such as plants, fragment pathways generated during sequencing analysis by NGS, or a reconstructed pathway generated by combining these fragment pathways. All kinds of these pathways have enzymes as a constituent element, and even in the case of the unknown pathways, at least a predicted enzyme gene predicted as an enzyme gene may be selected.

The predicted enzyme gene may be generally calculated as follows, but is not limited thereto.

First, NGS raw data is processed. That is, after DNA is extracted from a plant sample, raw data are obtained from an NGS-based sequencer (a sequencing determiner; a sequencer; for example, an Illumina HiSeq series equipment).

In general, NGS raw data processing is performed by a method of determining a sequencing having about 100 bp to 300 bp at both ends of a large amount of very small (usually about 300 bp to 5 kb) DNA fragments (pair-end library, mate-pair library).

The raw data is generally configured in the form of a text file, and for example, in the case of a device from Illumina Inc., the raw data is provided in a fastq format. However, other types of NGS data other than the device from Illumina Inc., can be used, and their data follows a unique format provided by the device depending on the situation. Here, the description is based on a fastq file, and a program may vary depending on the file format provided, but the process is substantially the same.

The first process goes through a process of removing a base pair indicating a low quality score based on quality scores of each base pair of the NGS raw data. In this process, for example, when the quality score is 30 points or less, cutoff is performed by filtering (Q30), and the filtering condition may be changed according to the condition and amount of data. Programs used in this process are, for example, Trimmomatic, fastQC, etc., and any related programs may be used. However, this step may be omitted depending on the situation.

In general, in order to decipher a genome, sequencing data of at least 150 times the length of the plant genome needs to be secured. However, since the amount of sequencing is proportional to cost, it is necessary to design the present invention to achieve the purpose of the present invention even if the amount of raw data is small in order to secure genomes for many plant species.

To this end, in the present invention, a relatively loose condition may be used to collect the results from a *de novo* assembly process to be described later in order to be able to operate at least (1/2 or less) the amount of input data.

In the next process, based on the filtered NGS raw data, a process of reconstructing the entire genome is performed in a similar way to a jigsaw puzzle. In other words, it is found whether a short sequencing of 100 bp to 300 bp obtained from small DNA fragments overlaps with other sequencings by using various algorithms, such as Smith-waterman algorithm or de *bruijn* graph algorithm, and it goes through the process of connecting and expanding the sequencings based thereon.

There are various sequencing assembly programs, but most of the programs that deal with NGS data recently are constructed using the de *bruijn* algorithm. The reason is that the amount of NGS data is enormous and the length of each sequencing is short, so if these are processed by the conventional method (Smith-waterman algorithm), it takes several months or more to go through the process.

Among the programs using the de *bruijn* algorithm, SOAPdenovo, which assembles plant genome data relatively well, is mainly used, and the process of reconstructing the genome may be performed. However, if necessary, other programs (for example, Velvet, AllPath-LG, ABySS, Platanus, etc.) may be used.

The result obtained through this process is a contig sequence in which the sequencing is connected as long as possible, and a scaffold sequence in which the sequence is determined using additional information (pair-end, mate-pair).

Based on the scaffold sequence, the gap closing process that determines the sequencing for the section that has not been determined (indicated by gap, N) in the corresponding sequencing is performed. In the process of the gap closing, GapCloser is mainly used, which is a program developed by the same team as the SOAP program. Through this process, many sections for which the sequencing has not been determined are reconstructed, and higher quality genomic data can be secured.

During this process, in order to obtain the maximum result with a small amount of data, the parameters of the SOAP program may be adjusted and operated in a range that allows even a small amount of data. In particular, after the assembly process is finished, the data of each scaffold sequence with a short length (100 bp to 1000 bp) are included without filtering to move to the next process, so that a relatively small amount of data may be obtained to obtain the desired result.

This strategy has the advantage of being able to obtain desired results at low cost in order to utilize the genome decoding and the results for a large number of plant species. The level of the genome decoding gets better as the cost increases, but the cost to go to a better level has a problem that increases exponentially, so it is important to determine the appropriate level and the optimized conditions at the low level allowed by the present invention are expected to be widely applied in the field where more data are usefully utilized from various viewpoints.

In the next process, the gene prediction is performed based on the sequencings obtained through the genome assembly process. Typically, the gene prediction is performed through a computer program rather than a human, and the sequencing of genes is secured as much as possible, and these are learned by computer programs and applied to the entire genome.

In the case of plant genomes, since there are many specific elements for each species, each time the genome is deciphered, the sequencing is determined in a large amount by extracting their RNA, and after performing the process of learning a computer program based on this data, the process of predicting genes is performed.

Here, the interesting thing is that when predicting genes of new plant genomes based on the pre-trained dataset from *Arabidopsis thaliana* which is the most studied in plants, a very large number of genes is predicted. For example, an average plant has less than 100,000 genes, and if the genomes of *Plantago depressa* are predicted from *Arabidopsis thaliana* data, 220,000 genomes are predicted. However, the interesting thing is that this predicted gene is not a fake. In other words, about 55% of these genes have intact a known functional domain, proving that this is not false information.

In this way, among programs that predict genes, for example, the AUGUSTUS program is used to perform an operation of predicting plant genes.

Finally, the enzyme prediction process is performed.

The predicted gene set is composed of a sequencing of each gene, an amino acid sequence resulting from translation of the sequencing, and location information of the gene. Among these data, a process of predicting whether there is a functional domain responsible for a key function of a gene based on the amino acid sequence and a process of predicting an enzyme gene involved in a pathway through comparison of additional data are performed.

First, the functional domain prediction uses a program called InterProScan, for example, to predict the presence or absence of over 30,000 defined functional domains. However, depending on the purpose, it is possible to use specialized programs such as PRINT, Pfam, etc. other than InterProScan.

In addition, through homologous gene search, data is secured using a BLAST program or corresponding programs to find genes with amino acid sequences similar to those of the already known enzyme genes.

By receiving the obtained functional domain and genes similar to the enzyme, the enzyme gene involved in the pathway is predicted finally.

In the case of the functional domains, genes with functional domains related to the action of enzymes (e.g., monooxygenase (Cytochrome P450); enzymes that move oxygen atoms) are selected as candidate genes, and in the case of finding similar genes, an operation of selecting genes that are very similar to enzyme genes with the known functions (e.g., e-value: 1e-10 or higher, amino acid similarity: 30% or higher).

In addition, it is preferable that the selected candidate genes are scored from the following equation, and the operation of determining their priority is also performed. There are many ways to score, and depending on the purpose, the equation can be modified as much as possible.

Enzyme gene prediction score

= {[Amino acid sequence similarity] * 0.5 ± log [Amino acid

sequence similarity statistical value (e-value, etc.)]/400

* 0.5}

+ {[Enzyme-related functional domain presence or absence

(0: none, 1: presence)]}

(Here, the score ranges from 0 to 2. The closer to 2, the higher the probability.) For the secured enzyme gene candidate target, additional predictive information may be calculated in consideration of a phylogenetic probability value and an expression probability value.

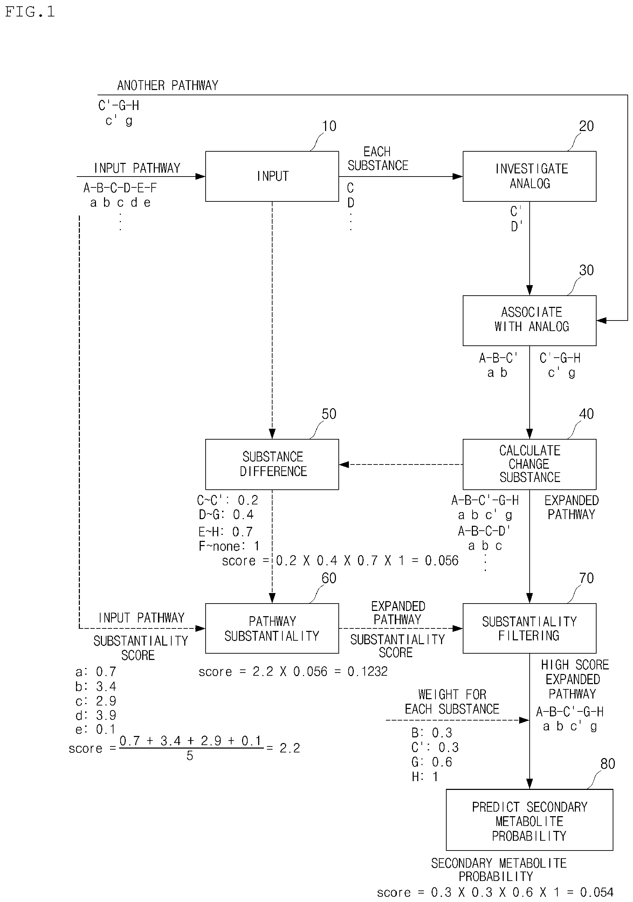

The predicted enzyme gene as described above may be mapped as an enzyme in each step of a known pathway and used to form a fragment pathway. If the final product of any fragment pathway and the starting substance of other fragment pathways are the same, by making these fragment pathways merge, a reconstruction pathway may be formed. In the input step of the present invention, not only a known pathway but also an unknown pathway may be input, and a fragment pathway composed of only a part or a reconstructed pathway formed by combining these fragment pathways may also be input. Various types of pathways are input to the apparatus of the present invention through the input module 10 as an input pathway. In the example of FIG. 1, a pathway through which a starting substance A, intermediate substances B, C, D, and E, and a terminating substance F as input pathways proceed to a next step by enzymes a, b, c, d, and e, respectively is illustrated.

The analog investigation step S20 is a step of investigating analogs having a similar function and a different structure to a substance of each step on the input pathway.

In general, the type of starting substances or terminating substances of the pathway is very diverse. For this reason, the case in which two or more pathways can be merged is extremely limited. Therefore, in the case of inferring based on the given pathway and combining a new pathway that may actually exist only by pathway reconstruction, the amount of the result may not be as large as expected.

However, due to the property of chemical substances, there are two cases in which the structure and function are classified. In one case, the structures of the chemical substances are quite different, but the functions thereof are the same, and the other case, on the contrary, the chemical substances have a similar chemical structure, but the functions thereof are completely different. The former case is called analog, for example, methanol has an analog as illustrated in FIG. 3. In the latter case, for example, enantiomers are examples, and there are cases in which the chemical formula is the same but the function is different due to structural differences. In particular, in the case of the enzymes, this enantiomer is strictly recognized and shows the property of reacting only to certain types of substances.

Using the properties of these chemical substances, the starting, intermediate, and terminating substances in the pathway are all investigated (if analogs are present).

The analog association step S30 is a step of establishing an association relationship between the analogs with respect to another pathway including the analog.

For the pathways investigated by analogs, the process of "expanding" the substances involved in the pathways based on these analogs is disclosed. In other words, when analogs that performs the same function as the starting substance and terminating substance of the found partial pathway are in different pathways, the operation of associating the starting substance and terminating substance (common analog substances) is performed. Here, the association is a connection concept of a relationship such as correspondence, equality, substitution, exchange, and identification between any substance or an analog thereof in any step on the first pathway and any substance or an analog thereof in any step on the second pathway, and includes both the situation in which the first pathway is the subject and the situation in which the second pathway is the subject, and the term is not limited thereto as long as the relationship is connected to become a crossroad of a chemical reaction path.

The change substance calculation step S40 is a step of performing a recalculation of a changed intermediate substance and a calculation of a new terminating substance, for a step after the step of establishing the association relationship.

After the association is made, since the intermediate substances are different in different pathways, an operation of calculating a new final product is performed by recalculating all of the chemical substances to be changed as illustrated in FIG. 4. Here, the calculation is a concept of representing an operation of predicting, simulating, judging, tracking, and outputting the chemical reaction between the substance before reaction and the enzyme and the substance after reaction with an enzyme in the pathway, taking into account the given environmental conditions such as temperature, pressure, and pH, and the term is not limited thereto as long as the reaction result is objectively produced.

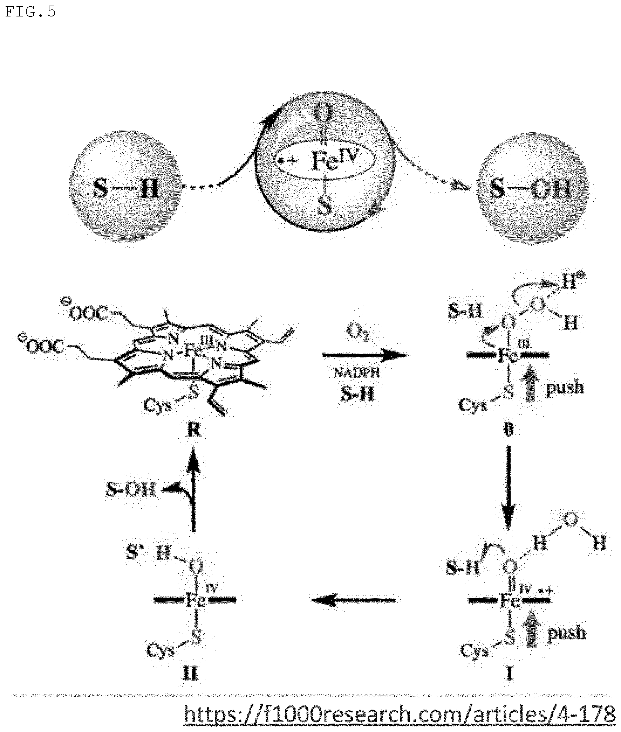

An example of recalculation of the biochemical synthesis process is as illustrated in FIG. 5.

For example, if the monooxygenase enzyme (Cytochrome P450) works, a new oxygen atom (O) is transferred to the starting substance (substrate), resulting in a final OH group.

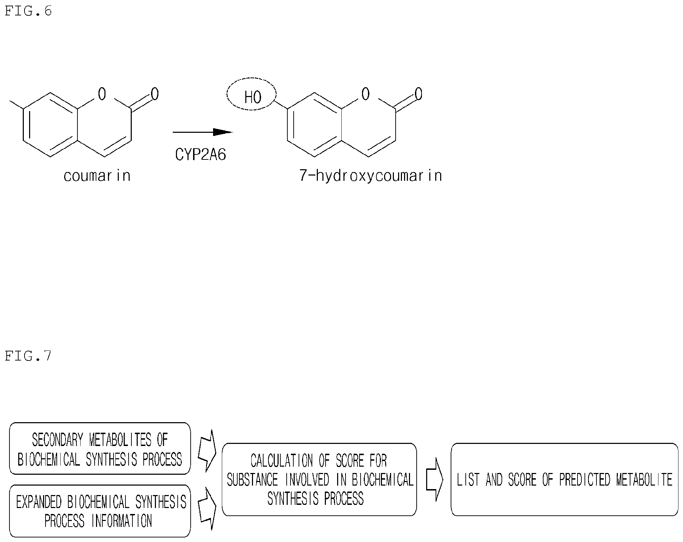

Therefore, as in the example of FIG. 6, a final product in which an OH group is added to the coumarin substance may be predicted.

Through this method, a new substance may be applied to the already known pathway, and the operation of calculating (tracking and predicting) substances that may be obtained therefrom may be performed.

The operation may be performed by referring to a database that records the properties of each enzyme, for example, InfoBoss Pathway Database.

<Example>

Referring to FIG. 1, the input pathway A-B-C-D-E-F (each step enzyme genes a, b, c, d, e) was input to the input module 10, and analogs were investigated for each substance on the input pathway, for example, substance C to find analog C'. And another pathway C'-G-H (each step enzyme gene c', g) containing the analog C' as a substance is searched. Then, the two pathways A-B-C-D-E-F (each step enzyme genes a, b, c, d, e) and C'-G-H (each step enzyme genes c', g) are associated with each other. Then, the changed substances such as the intermediate substance and the terminating substance changed after the associated step, i.e., the step having the analog C' are calculated. When all of the changed substances are calculated using a database storing the properties of the enzyme, etc., the expanded pathway AB-C'-GH (enzyme genes a, b, c', g at each stage) expanded in terms of substances for the input pathway is generated.

<Effect>

The expanded pathway is a pathway that is different from the given input pathway, and is a pathway that forms the association with the pathway that replaces analogs for the substance of each step on the input pathway, and has the analog among other pathways after the replaced step, and then is generated by calculating the change substances such as the intermediate substance, the terminating substance, and the like after the replaced step. Therefore, it is a new pathway in terms of pathways.

In other words, this expanded pathway expands the type of substances by the analog of the substance, and also expands the intermediate substance and the terminating substance, i.e., the secondary metabolite, and may further diversely infer the pathways that may be found from the plant samples. As a result, finally, it is possible not only to find a new type of pathway, but also to provide detailed information on the process of generating the final secondary metabolite at the same time.

By doing so, the binding properties of the given input pathways as the starting substance and the terminating substance themselves are limited, and even though the type of the final product, that is, the secondary metabolite as the terminating substance, is limited, there is an effect of expanding the connectivity between pathways in consideration of the analog of substances on the pathway.

<Calculation of Substance Difference between Pathways>

The input pathway and the expanded pathway may have a difference in the substance of each step on the pathway. However, there are cases where a quantitative indicator of how much difference occurs for each corresponding substance in each step is required. This is because the more the substances produced by the new expanded pathway are similar to those produced by the corresponding original input pathway, the higher the substantiality of the pathway. This quantitative indicator is called a substance difference. This substance difference itself may be an evaluation indicator for the expanded pathway, but, as will be described later, may also be used to calculate the substantiality score of the pathway for the expanded pathway.

In order to know the substance difference between the pathways, the method according to the present invention further includes a substance difference calculation step S50 in which the substance difference between the substance in each step in the input pathway and the substance in each corresponding step in the expanded pathway corresponding thereto is calculated.

On the other hand, the substance difference is defined as (1 - substance similarity), and the substance similarity is preferably a value of 0 to 1 represented by digitizing the similarity of substances.

In general, the substance difference can be defined in a number of ways, but in general, it is desirable to use a substance similarity that may be easily conceived. In other words, the substance difference may be considered as an opposite concept to the substance similarity. In other words, this operation digitizes the similarity of chemical substances (e.g., the difference in the number of molecules between the existing substance and the expandable substance in the analog phase, whether it is an enantiomer, etc.) obtained through literature research and converts the quantized value into a value (substance similarity between the chemical substances) between 0 and 1. Based on this substance similarity value, when the starting substance, the intermediate substance, and the terminating substance are changed, the substance difference between the chemical substances can be obtained as (1 - substance similarity between the chemical substances) from the substance similarity between the chemical substances possessed by each substance.

For example, in the example of FIG. 1, when the substance C is associated with the analog C', since the substance difference between the substances C and C' is 0.2, the substance difference between the substances D and G is 0.4, and the substance difference between the substances E and H is 0.7, and substance F has no corresponding substance, so when the substance difference is 1, the substance difference between the substances in this input pathway and the expanded pathway is 0.2 * 0.4 * 0.7 * 1 = 0.056.

<Substantiality Score of Input Pathway>

On the other hand, the input pathway may be a pathway that actually exists, but may be a fragment pathway generated in a DNA experiment or a theoretical reconstructed pathway generated by combining two or more pathways. Therefore, the substantiality of the expanded pathway derived from this input pathway depends on the degree of substantiality of the input pathway itself. Therefore, it is necessary to calculate the substantiality score of the input pathway.

It is preferable that the substantiality score of the input pathway is calculated according to the calculation of the fitness score of the enzyme gene in each step of the input pathway. That is, in the case of the enzyme gene that actually exists, a high score may be assigned, and in the case of an enzyme gene that may not actually exist, a score according to the likelihood of the absence may be assigned.

To this end, it is preferable that when there are a large number of enzymes mapped for each step of the input pathway, a maximum value among the fitness scores of the enzymes is selected, a sum of the fitness scores selected for all steps of the input pathway is calculated, and the substantiality score is calculated by an average value obtained by dividing the sum of the fitness scores by the number of steps of the input pathway.

For example, in the example of FIG. 1, when the fitness score of each enzyme gene of the input pathway is a: 0.7, b: 3.4, c: 2.9, d: 3.9, and e: 0.1, the substantiality score of this input pathway may be (0.7 + 3.4 + 2.9 + 3.9 + 0.1) / 5 = 2.2.

<Substantiality Score of Expanded pathway>

In the next step, an operation of calculating the 'expanded pathway substantiality score' from the 'input pathway information' and the 'predicted input pathway substantiality score' is performed. That is, it is preferable that the method further includes an expanded pathway substantiality scoring step S60 of calculating the substantiality score of the expanded pathway by determining the pathway substantiality of the expanded pathway based on the substantiality score of the input pathway and the substance difference of each step between the input pathway and the expanded pathway.

Specifically, for example, after obtaining the substance difference, it is possible to derive a value obtained by multiplying all substance difference values for substances in the entire pathway. The 'expanded pathway substantiality score' may be calculated by multiplying this value by the predicted score for each input pathway.

Through this process, it is possible to obtain all the information on new compounds with the possibility of synthesis through the identified input pathway and the analog expanded pathway. In addition, since the substances with this possibility may be various types, it is expected that additional inspection of the obtained substances will be required. For example, by checking whether the final product is actually a substance that has been identified in plants, it is possible to explore the possibility of whether the predicted secondary metabolite actually exists. Of course, this method has the disadvantage of not being able to identify a new, unknown secondary metabolite.

Finally, it is thought that the most reliable method is to confirm the predicted secondary metabolite by HPLC, GC, or the like.

For example, in the example of FIG. 1, since the substance difference between the input pathway and the substance of the expanded pathway is 0.056 and the substantiality score of the input pathway is 2.2, the substantiality score of the expanded pathway is 2.2 * 0.056 = 0.1232.

<Filtering High Score Expanded pathway>

On the other hand, the expanded pathway is an expansion of the received input pathway based on analog for each substance in each step, so a number of expanded pathways can be created for one input pathway, and the actual existence of each other may be high and low. Therefore, the filtering based on substantiality needs to be performed so that only the expanded pathways with high substantiality may be extracted and subsequent steps may be performed.

Therefore, the method further include a high-scoring expanded pathway extracting of extracting a high-scoring expanded pathway extraction step of extracting an expanded pathway with a high score higher than the reference value by substantially filtering the expanded pathway by the substantiality score of the expanded pathway indicating the probability that the expanded pathway actually exists.

<Calculation of Secondary Metabolite Actual Probability>

In addition, the terminating substance of the pathway, that is, the final substance is the secondary metabolite, and since it has a lot of utility in this secondary metabolite, it is a subject of much interest. However, it is not certain that the secondary metabolite produced by the expanded pathway is necessarily actually possible. This is because depending on the substance in each step on the expanded pathway, the secondary metabolite as the terminating substance may not actually be produced. For this reason, it is necessary to find out the probability indicating whether or not a secondary metabolite can actually be created.

Therefore, as illustrated in FIG. 7, the method further includes a secondary metabolite actual probability calculating step of calculating an actual probability of existence of a secondary metabolite which is a terminating substance by the expanded pathway by weighting and calculating each substance of each step in the expanded pathway.

This process refers to an operation of deriving final secondary metabolite information of expanded pathways based on the already known 'secondary metabolite of known pathway' and the extended 'expanded pathway information'. In addition, the information on the potential secondary metabolite substances among the intermediate substances to be obtained from the expanded pathway is also derived.

Each substance in the expanded pathway is weighted. For example, 1 point is assigned to the final product (terminating substance) of the expanded pathway, and 0.3 point is assigned to the intermediate products (intermediate substance), thereby calculating the probability that each substance may be prepared as a secondary metabolite (terminating substance). For reference, in the case of an intermediate products (intermediate substance), in general, since it is often passed directly to the next chemical reaction, it is less likely to be extracted as a secondary metabolite of a plant, and therefore, 0.3 points are assigned, but the specific value is not limited thereto.

However, for example, in the case of the already known secondary metabolite among intermediate products (intermediate substances), 0.6 points may be assigned. This is because in the process of integrating pathways, the already known secondary metabolites may be treated as the intermediate products.

In addition, the case in which the intermediate products (intermediate substances) overlap in the expanded pathway may also occur. In this case, the probability of the intermediate products (intermediate substance) is accumulated by the number of repetitions. For example, the intermediate products (intermediate substance) involved in two pathways may be assigned a score of 0.3 + 0.3 = 0.6.

Finally, if the probability of each substance exceeds 1, it is corrected to 1, so that the final probability has a value from 0 to 1.

For example, in the example of FIG. 1, when the weight of each substance of the expanded pathway AB-C'-G-H is B: 0.3, C': 0.3, G: 0.6, and H: 1, the actual probability of generating H as the secondary metabolite is predicted as 0.3 * 0.3 * 0.6 * 1 = 0.054.

If the substance with high probability is designated as a target for confirming the presence or absence of the actual substance among the obtained substances, it is possible to preferentially secure potential secondary metabolite based on data obtained from the genome of a specific plant.

<Apparatus>

Meanwhile, the apparatus of expansion of a biochemical pathway according to an embodiment of the present invention substantially corresponds to the method of the present invention described above, and includes the case of hard wiring in which each step of the method is formed as a hardware module. The apparatus according to the present invention includes an input module 10, an analog investigation module 20, an analog association module 30, and a change substance calculation module 40.

The input module 10 is a module that inputs an input pathway including a starting substance, an intermediate substance, and a terminating substance.

The analog investigation module 20 is a module that investigates analogs having a similar function and a different structure to a substance of each step on the input pathway.

The analog association module 30 is a module that establishes an association relationship between the analogs with respect to another pathway including the analog.

The change substance calculation module 40 is a module that performs a recalculation of a changed intermediate substance and a calculation of a new terminating substance, for a step after the step of establishing the association relationship.

<Program>

Meanwhile, a program of reconstruction of a biochemical pathway according to the present invention may be recorded in an information processing device readable storage medium to cause the information processing device to execute each step of the method described above.

Although the preferred embodiments of the present invention have been described above, the present invention is not limited to the disclosed embodiments, but can be implemented by changing into various different forms within the scope of the claims and the detailed description of the invention and the accompanying drawings. Only the embodiments are provided to ensure that the disclosure of the present invention is complete, and to fully inform the scope of the invention to those with ordinary knowledge in the technical field to which the present invention belongs. The present invention is defined by the scope of the claims.

**Claims**

1. A method of expansion of a biochemical pathway, comprising:

    inputting an input pathway including a starting substance, an intermediate substance, and a terminating substance;
    investigating analogs having a similar function and a different structure to a substance of each step on the input pathway;
    establishing an association relationship between the analogs for another pathway including the analogs; and
    performing a recalculation of a changed intermediate substance and a calculation of a new terminating substance, for a step after the establishing of the association relationship.

2. The method of claim 1, wherein an expanded pathway is generated by the calculation in the calculating of the changed substance, and
the method further comprising: extracting a high-scoring expanded pathway having a score higher than a reference value by substantially filtering the expanded pathway by a substantiality score of the expanded pathway indicating a probability that the expanded pathway actually exists.

3. The method of claim 1 or 2, wherein the expanded pathway is generated by the calculation in the calculating of the changed substance, and
the method further comprising: calculating an actual probability of existence of a secondary metabolite which is a terminating substance by the expanded pathway by weighting and calculating each substance of each step in the expanded pathway.

4. The method of claim 1 or 2, wherein the expanded pathway is generated by the calculation in the calculating of the changed substance,

    the method further comprising: calculating a substance difference between the substance of each step in the input pathway and the substance of each corresponding step in the expanded pathway corresponding thereto,
    the substance difference is defined as (1 - substance similarity), and
    the substance similarity is a value of 0 to 1 represented by digitizing the substance similarity.

5. The method of claim 1 or 2, wherein the expanded pathway is generated by the calculation in the calculating of the changed substance, and
the method further comprising: calculating the substantiality score of the expanded pathway by determining the pathway substantiality of the expanded pathway based on the substantiality score of the input pathway and the substance difference of each step between the input pathway and the expanded pathway.

6. The method of claim 5, wherein the substantiality score of the input pathway is calculated depending on the calculation of a fitness score of an enzyme gene in each step of the input pathway.

7. The method of claim 6, wherein when there are a large number of enzymes mapped for each step of the input pathway, a maximum value among the fitness scores of the enzymes is selected, a sum of the fitness scores selected for all steps of the input pathway is calculated, and the substantiality score is calculated by an average value obtained by dividing the sum of the fitness scores by the number of steps of the input pathway.

8. An apparatus of expansion of a biochemical pathway, comprising:

an input module configured to input an input pathway including a starting substance, an intermediate substance, and a terminating substance;

an analog investigation module configured to investigate analogs having a similar function and a different structure to a substance of each step on the input pathway;

an analog association module configured to establish an association relationship between the analogs for another pathway including the analogs; and

a change substance calculation module configured to perform a recalculation of a changed intermediate substance and a calculation of a new terminating substance, for a step after the establishing of the association relationship.

9. A program of expansion of a biochemical pathway recorded in an information processing device readable storage medium to cause the information processing device to execute each step of the method of claims 1 or 2.

FIG.1

ANOTHER PATHWAY
C'-G-H
c' g

INPUT PATHWAY
A-B-C-D-E-F
a b c d e
⋮

**10** INPUT

EACH SUBSTANCE
C
D
⋮

**20** INVESTIGATE ANALOG
C'
D'

**30** ASSOCIATE WITH ANALOG
A-B-C' | C'-G-H
a b | c' g

**50** SUBSTANCE DIFFERENCE
C~C': 0.2
D~G: 0.4
E~H: 0.7
F~none: 1
score = 0.2 X 0.4 X 0.7 X 1 = 0.056

**40** CALCULATE CHANGE SUBSTANCE
A-B-C'-G-H    EXPANDED PATHWAY
a b c' g
A-B-C-D'
a b c
⋮

**60** PATHWAY SUBSTANTIALITY

INPUT PATHWAY
SUBSTANTIALITY SCORE
a: 0.7
b: 3.4
c: 2.9
d: 3.9
e: 0.1
$$score = \frac{0.7 + 3.4 + 2.9 + 0.1}{5} = 2.2$$

score = 2.2 X 0.056 = 0.1232

EXPANDED PATHWAY
SUBSTANTIALITY SCORE

**70** SUBSTANTIALITY FILTERING

HIGH SCORE EXPANDED PATHWAY
A-B-C'-G-H
a b c' g

WEIGHT FOR EACH SUBSTANCE
B: 0.3
C': 0.3
G: 0.6
H: 1

**80** PREDICT SECONDARY METABOLITE PROBABILITY

SECONDARY METABOLITE PROBABILITY
score = 0.3 X 0.3 X 0.6 X 1 = 0.054

FIG.2

```
                        ┌─────────┐
                        │  START  │
                        └─────────┘
                             │
                             ▼                        S10
          ┌──────────────────────────────────────┐
          │   RECEIVE INPUT PATHWAY AND SCORE     │
          │    INPUT PATHWAY SUBSTANTIALITY       │
          └──────────────────────────────────────┘
                             │
                             ▼                        S20
          ┌──────────────────────────────────────┐
          │     INVESTIGATE ANALOG SUBSTANCE OF   │
          │       EACH STEP ON INPUT PATHWAY      │
          └──────────────────────────────────────┘
                             │
                             ▼                        S30
          ┌──────────────────────────────────────┐
          │     ASSOCIATE WITH ANALOG INCLUDED IN │
          │            ANOTHER PATHWAY            │
          └──────────────────────────────────────┘
                             │
                             ▼                        S40
          ┌──────────────────────────────────────┐
          │   RECALCULATE CHANGED INTERMEDIATE    │
          │     SUBSTANCE AND CALCULATE NEW       │
          │          TERMINATING SUBSTANCE        │
          └──────────────────────────────────────┘
                             │   EXPANDED PATHWAY
                             ▼                        S50
          ┌──────────────────────────────────────┐
          │       SCORE SUBSTANCE DIFFERENCE      │
          │   (= 1 - SUBSTANCE SIMILARITY) OF EACH│
          │    SUBSTANCE CHANGED BETWEEN INPUT    │
          │      PATHWAY AND EXPANDED PATHWAY     │
          └──────────────────────────────────────┘
                             │
                             ▼                        S60
          ┌──────────────────────────────────────┐
          │  SCORE EXPANDED PATHWAY SUBSTANTIALITY│
          │    (TERMINATING SUBSTANCE GENERATION  │
          │    POSSIBILITY) BY SUBSTANCE DIFFERENCE│
          │    AND INPUT PATHWAY SUBSTANTIALITY   │
          │                SCORE                  │
          └──────────────────────────────────────┘
                             │
                             ▼                        S70
          ┌──────────────────────────────────────┐
          │ PERFORM SUBSTANTIALITY FILTERING BY   │
          │   EXPANDED PATHWAY SUBSTANTIALITY TO  │
          │   SELECT HIGH SCORE EXPANDED PATHWAY  │
          └──────────────────────────────────────┘
                             │   HIGH SCORE
                             │   EXPANDED PATHWAY
                             ▼                        S80
          ┌──────────────────────────────────────┐
          │   CALCULATE PREDICTED SECONDARY       │
          │    METABOLITE (TERMINATING SUBSTANCE) │
          │   PROBABILITY BY WEIGHTING TERMINATING│
          │   SUBSTANCE AND INTERMEDIATE SUBSTANCE│
          │       OF HIGH SCORE EXPANDED PATHWAY  │
          └──────────────────────────────────────┘
                             │
                             ▼
                        ┌─────────┐
                        │   END   │
                        └─────────┘
```

FIG.3

**Alcohols**

Methanol

Silanol, a structural analog of methanol

Methanethiol, a structural analog of methanol

https://en.wikipedia.org/wiki/Structural_analog

FIG.4

SCORE FOR EACH PREDICTED BIOCHEMICAL SYNTHESIS PROCESS

RECONSTRUCTED BIOCHEMICAL SYNTHESIS PROCESS INFORMATION ⇒ EXPANSION OF BIOCHEMICAL SYNTHESIS PROCESS ⇒ EXPANDED BIOCHEMICAL SYNTHESIS PROCESS INFORMATION ⇒ EXPANDED BIOCHEMICAL SYNTHESIS INFORMATION SCORE CALCULATION ⇒ EXPANDED BIOCHEMICAL SYNTHESIS INFORMATION SCORE

FIG.5

https://f1000research.com/articles/4-178

FIG.6

coumarin      CYP2A6      7-hydroxycoumarin

FIG.7

| SECONDARY METABOLITES OF BIOCHEMICAL SYNTHESIS PROCESS | CALCULATION OF SCORE FOR SUBSTANCE INVOLVED IN BIOCHEMICAL SYNTHESIS PROCESS | LIST AND SCORE OF PREDICTED METABOLITE |
| --- | --- | --- |
| EXPANDED BIOCHEMICAL SYNTHESIS PROCESS INFORMATION | | |

Fig. 8

EP 4 134 961 A1

SRA database growth

50, 344, 196, 085, 946, 397 total bases
20, 775, 730, 396, 442, 001 open access bases

Total bases
Open access bases

02/5/2021 12:07am

FIG.9

xanthosine → 7-methylxanthosine synthase (EC 2.1.1.158) [AdoMet → AdoHcy] → 7-methylxanthosine → N-methyl nucleosidase (EC 3.2.2.25) [H₂O → D-ribose]

7-methylxanthine → theobromine synthase (EC 2.1.1.159) [AdoMet → AdoHcy] → 3,7-dimethylxanthine (theobromine) → caffeine synthase (EC 2.1.1.160) [AdoMet → AdoHcy] → 1,3,7-trimethylxanthine (caffeine)

https://en.wikipedia.org/wiki/Caffeine#/media/File:Caffeine_biosynthesis.tif

## Fig. 10

# Introduction (5)

**Fig. 2. Evolution of caffeine biosynthesis. (A)** The principal caffeine biosynthetic pathway. Three methylation steps are necessary to produce caffeine from xanthosine, involving the successive action of three NMTs: xanthosine methyltransferase (XMT), theobromine synthase [7-methylxanthine methyltransferase (MXMT)], and caffeine synthase [3,7-dimethylxanthine methyltransferase (DXMT)]. SAM, S-adenosylmethionine; SAH, S-adenosylhomocysteine. **(B)** Evolutionary position of caffeine-producing plants with respect to other eudicots (phylogeny adapted from www.mobot. org/MOBOT/research/APweb/). **(C)** ML phylogeny of coffee, tea, and cacao NMTs. Bootstrap support values (percentages) from 1000 replicates are shown next to relevant clades. Branch lengths are proportional to expected numbers of nucleotide substitutions per site. Colors identify genes assignable to the genomic blocks denoted in (D). **(D)** (Left) A model summarizing the duplication history of coffee NMT genes, following the phylogeny in (C). Three distinct tandem gene arrays evolved in situ on chromosome 1 from nearby gene duplicates (bold squares). The red and green blocks, colored as in (C), translocated (to chromo-

EP 4 134 961 A1

FIG.11

Path List    <show all path>

2 <show path> C16353 (R) C13747 (R) C07481
3 <show path> CX0003 (R) C16353 (R) C07480 (R) C07481
2 <show path> CX0009 (R) C07480 (R) C07481
1 <show path> CX0010 (R) C07481

CX0003 = > 7-Methylxanthine +
7-Methylxanthosine
(SECOND AND THIRD SUBSTANCES OF
KNOWN PATHWAY)

## INTERNATIONAL SEARCH REPORT

| International application No. |
| --- |
| **PCT/KR2020/017475** |

**A.    CLASSIFICATION OF SUBJECT MATTER**

**G16B 40/20**(2019.01)i; **G16B 30/10**(2019.01)i; **G16B 50/00**(2019.01)i

According to International Patent Classification (IPC) or to both national classification and IPC

**B.    FIELDS SEARCHED**

Minimum documentation searched (classification system followed by classification symbols)

G16B 40/20(2019.01); C12Q 1/68(2006.01); G06F 17/00(2006.01); G06F 19/00(2011.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 생화학 (biochemical), 경로 (pathway), 확장 (expension)

**C.    DOCUMENTS CONSIDERED TO BE RELEVANT**

| Category* | Citation of document, with indication, where appropriate, of the relevant passages | Relevant to claim No. |
| --- | --- | --- |
| A | KR 10-2016-0084275 A (SAMSUNG ELECTRONICS CO., LTD.) 13 July 2016 (2016-07-13)<br>See abstract, claim 1, and paragraph [0007]. | 1-9 |
| A | KR 10-2017-0049346 A (SAMSUNG ELECTRONICS CO., LTD.) 10 May 2017 (2017-05-10)<br>See entire document. | 1-9 |
| A | HOSSAIN, G. S. et al. Rewriting the metabolic blueprint: Advances in pathway diversification in microorganisms. Frontiers in Microbiology. 12 February 2018, vol. 9, document 155, pp. 1-10.<br>See entire document. | 1-9 |
| A | KR 10-2017-0095711 A (SAMSUNG ELECTRONICS CO., LTD.) 23 August 2017 (2017-08-23)<br>See entire document. | 1-9 |
| A | KR 10-2006-0098657 A (KOREA INSTITUTE OF SCIENCE AND TECHNOLOGY) 19 September 2006 (2006-09-19)<br>See entire document. | 1-9 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **15 March 2021** | **15 March 2021** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

INTERNATIONAL SEARCH REPORT
Information on patent family members

| Patent document cited in search report | | | Publication date (day/month/year) | Patent family member(s) | | | Publication date (day/month/year) |
|---|---|---|---|---|---|---|---|
| KR | 10-2016-0084275 | A | 13 July 2016 | None | | | |
| KR | 10-2017-0049346 | A | 10 May 2017 | US | 10497464 | B2 | 03 December 2019 |
| | | | | US | 2017-0121852 | A1 | 04 May 2017 |
| KR | 10-2017-0095711 | A | 23 August 2017 | US | 10510434 | B2 | 17 December 2019 |
| | | | | US | 2017-0235923 | A1 | 17 August 2017 |
| KR | 10-2006-0098657 | A | 19 September 2006 | US | 2006-0200330 | A1 | 07 September 2006 |

International application No.

**PCT/KR2020/017475**

Form PCT/ISA/210 (patent family annex) (July 2019)

## REFERENCES CITED IN THE DESCRIPTION

**Patent documents cited in the description**

- KR 101568399

- KR 1020170095711

**Non-patent literature cited in the description**

- **DENOEUD, F. ; CARRETERO-PAULET, L. ; DEREEPER, A. ; DROC, G. ; GUYOT, R. ; PIETRELLA, M. ; ZHENG, C. ; ALBERTI, A. ; ANTHONY, F. ; APREA, G.** The coffee genome provides insight into the convergent evolution of caffeine biosynthesis. *science,* 2014, vol. 345 (6201), 1181-1184